Europäisches Patentamt

European Patent Office

Office européen des brevets

(19)

(11) Veröffentlichungsnummer : **0 059 265**
**B1**

(12) **EUROPÄISCHE PATENTSCHRIFT**

(45) Veröffentlichungstag der Patentschrift :
28.08.85

(51) Int. Cl.⁴ : **A 61 L 15/03, A 61 K 35/16**

(21) Anmeldenummer : 81110421.5

(22) Anmeldetag : 14.12.81

(54) **Material zum Abdichten und Heilen von Wunden und Verfahren zu dessen Herstellung.**

(30) Priorität : 16.02.81 DE 3105624

(43) Veröffentlichungstag der Anmeldung :
08.09.82 Patentblatt 82/36

(45) Bekanntmachung des Hinweises auf die Patenterteilung : 28.08.85 Patentblatt 85/35

(84) Benannte Vertragsstaaten :
**AT BE CH DE FR GB IT LI LU NL SE**

(56) Entgegenhaltungen :
DE-A- 2 914 822
DE-A- 2 914 822
GB-A- 704 517
GB-A- 704 517
CHEMICAL ABSTRACTS, Band 94, Nr. 2, 12. Januar
1981, Seite 246, Nr. 7649t, Columbus, Ohio, USA
CHEMICAL ABSTRACTS, Band 94, Nr. 2, 12. Januar
1981, Seite 246, Nr. 7649t, Columbus, Ohio, USA, A.
STEMBERGER et al.: "Biochemical and physiological
aspects of fibrin adhesion"
Blut, Band 42, Heft Nr. 2, Februar 1981, Abstract 42

(73) Patentinhaber : **Hormon-Chemie München GmbH**
**Freisinger Landstrasse 74**
**D-8000 München 45 (DE)**

(72) Erfinder : **Zimmermann, Eberhard, Prof. Dr.**
**Am Braaken 16**
**D-4400 Münster (DE)**
Erfinder : **Schiele, Ulrich, Dr.**
**Schwedenstrasse 68**
**D-8000 München (DE)**

(74) Vertreter : **Kunz, Ekkehard et al**
**Chemie Linz AG Patentabteilung St. Peter-Strasse 25**
**A-4020 Linz (AT)**

## Beschreibung

Die Erfindung betrifft ein Material zum Abdichten und Heilen von Wunden, bestehend aus einem Kollagenträger, einer Fibrinogenkomponente und einer Thrombinkomponente, sowie ein Verfahren zur Herstellung des Materials.

Es ist bekannt, Kollagen, welches ein wesentliches Protein des Bindegewebes darstellt, zur Wundbehandlung zu verwenden. Kollagen kann z. B. aus Tierhäuten und Sehnen mittels physikalischer und chemischer Methoden abgetrennt und modifiziert werden und als Kollagenblatt, -vlies oder -schaum auf eine Wunde appliziert werden (DE-AS 16 17 780).

Es ist weiters bekannt, eine lokale Blutstillung und Gewebeklebung mittels Blutgerinnungsfaktoren, wie Fibrinogen, Thrombin und Blutgerinnungsfaktor XIII zu erreichen.

Auch die Kombination von Fibrinogen und Kollagen zur Blutstillung in der Herzchirurgie ist bereits in Wien. med. Wschr. 7, 86 bis 89 (1976), beschrieben. Allerdings ist die Anwendung zeit- und materialaufwendig : Gefriergetrocknetes Human-Fibrinogen wird auf 37 °C erwärmt, auf ein Kollagenvlies aufgebracht und dort durch den Zusatz einer wäßrigen Lösung von Thrombin und einer wäßrigen Lösung von Faktor XIII zur Gerinnung gebracht, worauf das Kollagen mit der mit dem so gebildeten Fibrin beschichteten Seite auf die blutende Stelle aufgepreßt wird. Es ist jedoch schwierig, den richtigen Zeitpunkt zur Übertragung auf die Wunde zu treffen. Überträgt man den Ansatz zu früh, so verfließen die Gerinnungsfaktoren in Bereiche, in denen sie nicht erwünscht sind, wie z. B. in Blutgefäße, überträgt man den Ansatz zu spät, so findet keine ausreichende Verklebung mehr statt. Um bei einer Operation auf unerwartet auftretende Blutungen reagieren zu können, muß immer eine ausreichend große Menge an von fibringetränktem Kollagen vorbereitet sein, das dann oft nicht verwendet wird und verworfen werden muß.

Auch das in DE-OS 29 14 822 beschriebene Material zum Heilen von Wunden, welches Blutkoagulationsfaktor XIII und Thrombin an sich fixiert aufweist, kann dieses Problem nicht lösen, da das zur Blutgerinnung ebenfalls notwendige Fibrinogen nicht in dem Material enthalten ist, sodaß das Material z. B. für eine Verbrauchskoagulopathie nicht geeignet ist.

Aus Chemical Abstracts 94 (1981) 7649t ist bekannt, daß eine Kombination von Thrombin mit Faktor XIII beständig und 162 Tage ohne Aktivitätsverlust lagerfähig ist, während ein Fibrinkleber, enthaltend Fibrinogen, Plasminogen und Faktor XIII, durch die Zugabe von Thrombin und CaCl₂ ein Gerinnsel bildet und sich zusammen mit einem Kollagenschwamm gut zur Wundverklebung eignet. Fibrinkleber, enthaltend Fibrinogen und Thrombin in lagerfähiger Form nebeneinander, sind nicht beschrieben.

Überraschenderweise konnte nun ein Material gefunden werden, das sämtliche für die Blutgerinnung nötigen Bestandteile nebeneinander enthält, die aber erst im Gebrauchsfall miteinander reagieren, sodaß dieses Material über längere Zeit in gebrauchsfertigem Zustand lagerfähig ist. Das ist dann möglich, wenn die für die Blutgerinnung nötigen Faktoren auf einem Kollagenträger in Gegenwart eines zumindest vorwiegend aus einem organischen Lösungsmittel bestehenden Mediums aufgebracht werden, wobei dann die Bestandteile überraschenderweise gut auf dem Kollagenträger haften bleiben, obwohl die Fibrinbildung noch nicht oder zumindest nicht in nennenswertem Umfang eingesetzt hat.

Gegenstand der vorliegenden Erfindung ist demnach ein Material zum Abdichten und Heilen von Wunden, bestehend im wesentlichen aus Kollagen und die Blutgerinnung bewirkenden Substanzen, erhältlich durch einseitiges oder allseitiges Beschichten eines Kollagenträgers in Gegenwart eines organischen Lösungsmittels, das geringe Mengen an Wasser enthalten kann, mit einer Mischung einer Fibrinogenkomponente, enthaltend Fibrinogen, den Faktor XIII enthaltendes Fibrinogen oder Mischungen derselben, und mit einer Thrombinkomponente, enthaltend Thrombin, in Gegenwart von Körperflüssigkeit thrombinfreisetzende Substanzen oder Mischungen derselben, die außerdem die üblichen Zusätze, wie Calciumionen, Proteaseinhibitoren, Heparinantagonisten, die Einsprossung und das Wachstum von Fibroplasten fördernde Substanzen, wie Fibronectin, sowie infektionshemmende Arzneimittel enthalten kann und anschließendes Verdampfen des Lösungsmittels.

Zur Herstellung des erfindungsgemäßen Materials können verschiedene Typen von Kollagen verwendet werden, wie natives Kollagen oder chemisch modifiziertes Kollagen, wie z. B. quervernetztes Kollagen, verestertes Kollagen oder Kollagen mit modifizierten Aminogruppen.

Der Kollagenträger kann in Form von Schaum, Vlies oder in Form eines Filmes verwendet werden, wobei der Kollagenschaum besonders bevorzugt ist.

Als Fibrinogenkomponente kann eingesetzt werden : Tierisches oder menschliches Fibrinogen, zweckmäßig in einer Menge von 0,05 bis 20 mg/cm², wobei der Bereich von 0,5 bis 5 mg/cm² besonders bevorzugt ist. Das Fibrinogen kann hoch gereinigt sein, geringe Mengen an Gerinnungsfaktor XIII enthalten oder auch Gerinnungsfaktor XIII angereichert enthalten. Üblicherweise wird man Fibrinogen mit einem Gehalt von 0,5 bis 20 U/cm², vorzugsweise mit einem Gehalt von 1 bis 10 U/cm² an Gerinnungsfaktor XIII verwenden. Der Gerinnungsfaktor XIII kann auch gesondert zugesetzt werden. Das Fibrinogen kann in kristalliner oder amorpher Form oder als Lyophylisat eingesetzt werden.

Die Thrombinkomponente kann tierischen oder menschlichen Ursprungs sein und zweckmäßig in einer Menge von 1 μg bis 5 mg/cm² eingesetzt werden, wobei der Bereich von 50 μg bis 1 mg/cm² bevorzugt ist. Es ist auch möglich, eine Kombination von Faktoren, die Thrombin freisetzen, zu verwenden. Solche Faktoren sind beispielsweise Prothrombin und Gerinnungsfaktor Xa.

Neben den Gerinnungsfaktoren können die üblichen, den Blutgerinnungsvorgang und die Wundheilung beeinflussenden Substanzen auf den Kollagenträger aufgebracht werden. Besonders vorteilhaft ist es, Proteaseinhibitoren, wie z. B. Aprotinin (1 bis 1 000 U/cm²) sowie Heparin-Antagonisten, wie z. B. Protaminchlorid (0,01 bis 5 mg/cm²) oder Faktoren, die die Einsprossung und das Wachstums von Fibroplasten fördern und damit die Wundheilung beschleunigen, wie z. B. Fibronectin, auf das Kollagen aufgebracht werden. Ebenson können Calciumionen, etwa über Calciumchlorid, in einer Menge von 2 nMol bis 2 μMol/cm² mit verwendet werden.

Das erfindungsgemäße Mittel kann auch infektionshemmende Arzneimittel, wie Bakterizide, enthalten.

Zur Kennzeichnung der beschichteten Seite des erfindungsgemäßen Mittels ist es auch möglich, den aufzubringenden Substanzen einen geeigneten Farbstoff, wie z. B. Hämin, beizumischen.

Das wesentliche Ziel der Erfindung ist das Nebeneinander-Vorhandensein von Fibrinogenpartikeln und Thrombin- bzw. thrombinfreisetzenden Partikeln auf einen Kollagenträger, ohne daß sie miteinander reagieren. Dies kann erreicht werden, indem Fibrinogen- und Thrombin- bzw. thrombinfreisetzende Partikel als Kristalle, als Lyophylisate oder in amorpher Form mit einem organischen Lösungsmittel versetzt werden, worauf durch inniges Vermischen etwa in einem hochtourigen Mixer, gegebenenfalls unter Zerkleinerung großer Kristalle, eine Suspension gebildet wird. Dies kann für beide Gerinnungsfaktoren gesondert oder in einem einzigen Arbeitsgang durchgeführt werden. Auch die übrigen, die Gerinnung und Wundheilung beeinflussenden Faktoren, Ionen oder Arzneimittel können bereits in dem Lösungsmittel mit suspendiert oder gelöst werden. Hierauf wird die Suspension einseitig oder allseitig auf den Kollagenträger durch Bestreichen, Besprühen oder Eintauchen aufgetragen und das Lösungsmittel bei Raumtemperatur oder unter Kühlung, bei Normaldruck oder unter Anlegen eines Vakuums verdunsten gelassen. Die Fibrinogen- und Thrombinpartiekel bleiben auf der Kollagenoberfläche haften.

Zur Suspendierung der Gerinnungsfaktoren kann eine Vielzahl organischer Lösungsmittel verwendet werden. Die Lösungsmittel, sie können geringe Mengen von Wasser enthalten, sollen ausreichend flüchtig sein und die Gerinnungsfaktoren nicht inaktivieren. Solche Lösungs- bzw. Suspensionsmittel sind beispielsweise niedere geradkettige oder verzweigte Alkohole von $C_1$ bis $C_5$, insbesondere n-Propanol, Isopropanol, n-Butanol, Isobutanol und Äthanol, Ketone, wie z. B. Aceton oder Methyläthylketon, aliphatische oder cycloaliphatische Äther, wie z. B. Dimethyl- oder Diäthyläther, Tetrahydrofuran oder Dioxan, Ester, wie z. B. Essigsäureäthylester, Nitrile, wie Acetonitril, oder aliphatische halogenierte Kohlenwasserstoffe, wie z. B. Tetrachlorkohlenstoff, Methylenchlorid oder Chloroform.

Eine weitere Möglichkeit, das erfindungsgemäße Material herzustellen, besteht darin, den Kollagenträger mit einem der für die Suspendierung geeigneten Lösungsmittel, das geringe Mengen an Wasser enthalten kann, zu befeuchten und die Fibrinogen- und Thrombinkomponente sowie die Hilfsstoffe gleichzeitig oder nacheinander in fester Form gleichmäßig auf die befeuchtete Kollagenschicht aufzutragen und das Lösungsmittel verdunsten zu lassen. Auch hier bleiben die Partikel fest and der Oberfläche haften.

Als eine Abänderung des Verfahrens ist es möglich, den Kollagenträger mit einer sehr geringen Menge an Wasser zu befeuchten, die gerade ausreicht, um die Fibrinogen- und Thrombinpartikel auf der Oberfläche des Kollagenträgers zu fixieren.

Die Beschichtung des Kollagenträgers kann einseitig oder allseitig erfolgen. Die einseitige Beschichtung auf der später der Wunde zugekehrten Seite ist vorteilhaft zum Verschließen von Operationswunden, da so die Verklebung nur an der zu verklebenden Wunde erfolgt, während das Verwachsen der inneren Wunde mit dem gegenüberliegenden Gewebe verhindert wird. Wird das erfindungsgemäße Material hingegen zum Verschließen und Heilen eines Hohlraumes verwendet, so kann ein entsprechend geformtes Stück Kollagenschaum in die Suspension der Gerinnungsfaktoren getaucht werden, sodaß eine allseitige Beschichtung erreicht wird.

Gegenüber der bisher bekannten Kombination von Fibrinklebung auf wäßriger Basis und Kollagen bietet das erfindungsgemäße Mittel beträchtliche Vorteile :

Da die Fibrinogenkomponente und die Thrombinkomponente mit Hilfe eines organischen Lösungsmittels, also weitgehend in Abwesenheit von Wasser auf den Kollagenträger aufgebracht wurden, lösen sie sich erst dann und bilden Fibrin, wenn seröse Flüssigkeit oder Blut hinzutritt. Die Fibrinbildung erfolgt also genau zum richten Zeitpunkt am richtigen Ort. Auch in jenem Fall, in dem der Kollagenträger mit einer sehr geringen Menge an Wasser befeuchtet und anschließend mit den festen Fibrinogen- und Thrombinpartikeln versetzt wird, findet keine nennenswerte Fibrinbildung statt, da das Wasser nur als Bindemittel zwischen dem Kollagenträger und den einzelnen Partikeln der Gerinnungsfaktoren dient.

Die Handhabung des erfindungsgemäßen Materials ist sehr einfach. Es kann trocken eingesetzt werden, klebt daher nicht an Operationshandschuhen und -besteck und hat eine günstige, elastisch verformbare Konsistenz. Die Verklebung erfolgt erst auf der Wunde. Da sich das Fibrin nur im Kollagenträger bildet, können auch heterologe, also nicht vom Menschen stammende Gerinnungsfaktoren eingesetzt werden. Dies hat den besonderen Vorteil, daß die Gefahr der Übertragung von Virus-Hepatitis ausgeschlossen werden kann.

Auch die Lagerung des erfindungsgemäßen Materials ist einfach. Es wird bei Kühlschranktemperatur oder Raumtemperatur unter Ausschluß von Feuchtigkeit etwa durch Einschweißen in eine Folie steril aufbewahrt.

Das Mittel eignet sich für alle Arten von Wundbehandlung und Wundheilung. Es dient insbesondere zum Abdichten und Verkleben von inneren und äußeren Wunden, zur Sicherung von Operationsnähten, zur Heilung flächiger Wunden oder von Wundhohlräumen. Besonders eignet es sich auch für operativ oder traumatisch bedingte, große oder kleine Knochenhöhlen, innerhalb derer die Stillung von Blutungen oft sehr problematisch ist, z. B. nach Zahnextraktionen, Operationen im Bereich der Otologie oder Frakturen.

Beispiel 1

1 000 mg Fibrinogen, Faktor XIII enthaltend (vom Rind), 25 mg Thrombin (vom Rind), 5 mg $CaCl_2 \times 2H_2O$, 250 000 Einheiten Aprotinin und 10 mg Protamin (z. B. als Chlorid) werden in einem schmalen und hohen Gefäß unter Kühlung mit so viel gekühltem Äthanol versetzt, daß die Substanzen mit Flüssigkeit bedeckt sind. Dann wird 30 sec. mit Hilfe einer Ultra-Turrax-Apparatur homogenisiert. Die Suspension wird mit einer Sprühapparatur auf 500 $cm^2$ Kollagenschaum aufgebracht. Das Äthanol wird verdunsten gelassen. Die Partikel bleiben auf der Oberfläche des Kollagenschaumes haften.

Beispiel 2

1 000 mg Fibrinogen, Faktor XIII enthaltend (vom Rind), werden in einem schmalen und hohen Gefäß mit so viel n-Propanol versetzt, daß die Substanz mit Flüssigkeit bedeckt ist. Dann wird 60 sec. mit Hilfe einer Ultra-Turrax-Apparatur homogenisiert. 50 mg Thrombin (vom Rind) werden in einem schmalen, hohen Gefäß mit so viel n-Propanol versetzt, daß die Substanz mit Flüssigkeit bedeckt ist. Dann wird 10 sec. mit Hilfe einer Ultra-Turrax-Apparatur homogenisiert.

Die beiden Suspensionen werden vereinigt und mit einer Sprühapparatur auf 500 $cm^2$ eines Kollagenfilms aufgebracht. Das n-Propanol wird unter Vakuum abgedampft. Die Fibrinogen- und Thrombinpartiekel bleiben auf der Kollagenoberfläche haften.

Beispiel 3

1 500 mg Fibrinogen, Faktor XIII enthaltend (vom Rind), 50 mg Thrombin (vom Rind) und 10 mg Protamin (als Chlorid) werden in einem schmalen und hohen Gefäß unter Kühlung mit so viel gekühltem Tetrachlorkohlenstoff versetzt, daß die Substanzen mit Flüssigkeit bedeckt sind. Dann wird 30 sec. mit Hilfe einer Ultra-Turrax-Apparatur homogenisiert. Die Suspension wird mit einer Sprühapparatur auf 500 $cm^2$ Kollagenvlies aufgebracht. Der Tetrachlorkohlen stoff wird verdunsten gelassen. Die Partikel von Fibrinogen, Thrombin und Protaminchlorid bleiben auf der Oberfläche des Kollagenvlieses haften.

Beispiel 4

500 $cm^2$ Kollagenschaum werden mit Essigsäureäthylester besprüht, bis die Oberfläche gerade befeuchtet ist. Darauf wird eine Mischung der folgenden, in fester Form zermahlenen Substanzen gleichmäßig verteilt : 1 000 mg Fibrinogen, Faktor XIII enthaltend (vom Rind), 25 mg Thrombin (vom Rind), 5 mg $CaCl_2 \times 2H_2O$, 250 000 Einheiten Aprotinin und 10 mg Protamin (als Chlorid). Der Essigsäureäthylester wird verdunsten gelassen. Die Partikel bleiben auf der Oberfläche des Kollagenschaumes haften.

Beispiel 5

1 000 mg menschliches Fibrinogen, Faktor XIII enthaltend, 30 mg menschliches Thrombin + 10 mg Protamin (als Chlorid) werden in einem schmalen, hohen Gefäß unter Kühlung mit so viel n-Butanol von 0 bis 4 °C versetzt, daß die Substanzen mit Flüssigkeit überdeckt sind. Dann wird 30 sec. mit Hilfe einer Ultra-Turrax-Apparatur homogenisiert. Die Suspension wird mit einer Sprühapparatur auf 500 $cm^2$ Kollagenschaum aufgebracht. Das n-Butanol wird im Vakuum abgedampft. Die Partikel bleiben auf der Oberfläche des Kollagenschaumes haften.

Beispiel 6

500 mg Fibrinogen, Faktor XIII enthaltend (vom Rind), 25 mg Thrombin (vom Rind), 10 mg Protamin (als Chlorid) werden in einem schmalen, hohen Gefäß unter Kühlung mit so viel Äthanol von 0 bis 4 °C versetzt, daß die Substanzen mit Flüssigkeit gerade überdeckt sind. Dann wird 30 sec. homogenisiert. In diese Suspension werden kegelförmig zugeschnittene, zur Ausstopfung von Zahnextraktionswunden geeignete Kollagenschaumstücke getaucht. Das Äthanol wird verdunsten gelassen. Die Partikel bleiben auf der Kollagenoberfläche haften.

Beispiel 7

500 mg Fibrinogen (vom Rind), 25 mg Thrombin (vom Rind), 1 000 Einheiten Faktor XIII sowie 5 mg Protamin (als Chlorid) werden unter Kühlung mit so viel Acetonitril versetzt, daß die Substanzen mit Flüssigkeit bedeckt sind. Nach Homogenisieren wird die Suspension in ein Gefäß mit einem variablen Austrittsschlitz in der Art einer Dünnschichtstreichmaschine gegeben, gleichmäßig auf 500 cm² Kollagenschaum aufgegeben und das Lösungsmittel verdunsten gelassen. Die aufgebrachten Partikel bleiben auf der Oberfläche haften.

Beispiel 8

500 cm² Kollagenschaum werden mit Hilfe einer Sprühapparatur mit $H_2O$ besprüht, sodaß 1 mg $H_2O$ auf 1 cm² kommt. Das Wasser wird sofort von der Oberfläche des Kollagenschaumes aufgenommen, ohne daß die Makrostruktur des Kollagens verändert wird. Auf die nun klebrige Oberfläche werden 500 mg Fibrinogen vom Rind, 20 mg Thrombin (vom Rind), 200 000 U/cm² Aprotinin sowie 5 mg Protamin (als Chlorid) in feinen Partikeln aufgebracht. Nach kurzer Lagerung an der Luft verliert die Kollagenoberfläche die klebrige Konsistenz. Die aufgebrachten Partikel bleiben auf der Oberfläche haften.

**Patentansprüche** (für die Vertragsstaaten : BE, CH, DE, FR, GB, IT, LI, LU, NL, SE)

1. Material zum Abdichten und Heilen von Wunden, bestehend im wesentlichen aus Kollagen und die Blutgerinnung bewirkenden Substanzen, erhältlich durch einseitiges oder allseitiges Beschichten eines Kollagenträgers in Gegenwart eines organischen Lösungsmittels, das geringe Mengen an Wasser enthalten kann, mit einer Mischung einer Fibrinogenkomponente, enthaltend Fibrinogen, den Faktor XIII enthaltendes Fibrinogen oder Mischungen derselben, und mit einer Thrombinkomponente, enthaltend Thrombin, in Gegenwart von Körperflüssigkeit thrombinfreisetzende Substanzen oder Mischungen derselben, die außerdem die üblichen Zusätze, wie Calciumionen, Proteaseinhibitoren, Heparinantagonisten, die Einsprossung und das Wachstum von Fibroplasten fördernde Substanzen, wie Fibronectin, sowie infektionshemmende Arzneimittel enthalten kann und anschließendes Verdampfen des Lösungsmittels.

2. Material zum Abdichten und Heilen von Wunden, bestehend im wesentlichen aus Kollagen und die Blutgerinnung bewirkenden Substanzen, erhältlich durch einseitiges oder allseitiges Beschichten eines mit einer geringen Menge an Wasser befeuchteten Kollagenträgers mit einer Mischung einer festen Fibrinogenkomponente, enthaltend Fibrinogen, den Faktor XIII enthaltendes Fibrinogen oder Mischungen derselben, einer festen Thrombinkomponente, enthaltend Thrombin, in Gegenwart von Körperflüssigkeit thrombinfreisetzende Substanzen oder Mischungen derselben, die außerdem die üblichen Zusätze, wie Calciumionen, Proteaseinhibitoren, Heparinantagonisten, die Einsprossung und das Wachstums von Fibroplasten fördernde Substanzen, wie Fibronectin, sowie infektionshemmende Arzneimittel enthalten kann.

3. Material nach den Ansprüchen 1 und 2, dadurch gekennzeichnet, daß der Kollagenträger ein Kollagenschaum ist.

4. Material nach den Ansprüchen 1 bis 3, dadurch gekennzeichnet, daß es Fibrinogenpartikel in einer Menge von 0,05 bis 20 mg/cm² enthält.

5. Material nach Anspruch 4, dadurch gekennzeichnet, daß es Fibrinogenpartikel in einer Menge von 0,5 bis 5 mg/cm² enthält.

6. Material nach den Ansprüchen 1 bis 5, dadurch gekennzeichnet, daß es Thrombin oder thrombinfreisetzende Partikel in einer Menge von 1 µg bis 5 mg/cm² enthält.

7. Material nach Anspruch 6, dadurch gekennzeichnet, daß es Thrombin oder thrombinfreisetzende Partikel in einer Menge von 50 µg bis 1 mg/cm² enthält.

8. Verfahren zur Herstellung eines Materials zum Heilen und Abdichten von Wunden nach den Ansprüchen 1,3,4,5,6 und 7, dadurch gekennzeichnet, daß ein Kollagenträger einseitig oder allseitig in Gegenwart eines organischen Lösungsmittels, das geringe Mengen an Wasser enthalten kann, mit einer Mischung einer Fibrinogenkomponente, enthaltend Fibrinogen, den Faktor XIII enthaltendes Fibrinogen oder Mischungen derselben, und mit einer Thrombinkomponente, enthaltend Thrombin, in Gegenwart von Körperflüssigkeit thrombinfreisetzende Substanzen oder Mischungen derselben, die außerdem die üblichen Zusätze, wie Calciumionen, Proteaseinhibitoren, Heparinantagonisten, die Einsprossung und das Wachstum von Fibroplasten fördernde Substanzen, wie Fibronectin, sowie infektionshemmende Arzneimittel enthalten kann, beschichtet und anschließend das Lösungsmittel verdampft wird.

9. Verfahren zur Herstellung eines Materials zum Heilen und Abdichten von Wunden nach den Ansprüchen 2 bis 7, dadurch gekennzeichnet, daß ein mit einer geringen Menge an Wasser befeuchteter Kollagenträger einseitig oder allseitig mit einer Mischung einer festen Fibrinogenkomponente, enthaltend Fibrinogen, den Faktor XIII enthaltendes Fibrinogen oder Mischungen derselben, einer festen Thrombinkomponente, enthaltend Thrombin, in Gegenwart von Körperflüssigkeit thrombinfreisetzende Substanzen oder Mischungen derselben, die außerdem die üblichen Zusätze, wie Calciumionen, Proteaseinhibitoren, Heparinantagonisten, die Einsprossung und das Wachstum von Fibroplasten fördernde Substanzen, wie Fibronectin, sowie infektionshemmende Arzneimittel enthalten kann, beschichtet wird.

10. Verfahren nach Anspruch 8, dadurch gekennzeichnet, daß die Fibrinogenkomponente und die Thrombinkomponente einzeln oder gemeinsam in einem organischen Lösungsmittel, das geringe Mengen an Wasser enthalten kann, suspendiert, nötigenfalls gemischt, auf eine oder beide Oberflächen des Kollagenträgers aufgetragen werden, worauf das Lösungsmittel verdampft wird.

11. Verfahren nach Anspruch 10, dadurch gekennzeichnet, daß die Suspension aufgesprüht wird.

**Patentansprüche** (für den Vertragsstaat AT)

1. Verfahren zur Herstellung eines Verbandmaterials, enthaltend Kollagen und die Blutgerinnung bewirkende Substanzen, dadurch gekennzeichnet, daß ein Kollagenträger in Gegenwart eines organischen Lösungsmittels, das geringe Mengen an Wasser enthalten kann, einseitig oder allseitig mit einer Mischung einer Fibrinogenkomponente, enthaltend Fibrinogen, den Faktor XIII enthaltendes Fibrinogen oder Mischungen derselben, mit einer Thrombinkomponente, enthaltend Thrombin, in Gegenwart von Körperflüssigkeit thrombinfreisetzende Substanzen oder Mischungen derselben, die außerdem die üblichen Zusätze, wie Calciumionen, Proteaseinhibitoren, Heparinantagonisten, die Einsprossung und das Wachstum von Fibroplasten fördernde Substanzen, wie Fibronectin, sowie infektionshemmende Arzneimittel enthalten kann, beschichtet und anschließend das Lösungsmittel verdampft wird.

2. Verfahren zur Herstellung eines Verbandmaterials, enthaltend Kollagen und die Blutgerinnung bewirkende Substanzen, dadurch gekennzeichnet, daß ein mit einer geringen Menge an Wasser befeuchteter Kollagenträger einseitig oder allseitig mit einer Mischung einer festen Fibrinogenkomponente, enthaltend Fibrinogen, den Faktor XIII enthaltendes Fibrinogen oder Mischungen derselben, einer festen Thrombinkomponente, enthaltend Thrombin, in Gegenwart von Körperflüssigkeit thrombinfreisetzende Substanzen oder Mischungen derselben, die außerdem die üblichen Zusätze, wie Calciumionen, Proteaseinhibitoren, Heparinantagonisten, die Einsprossung und das Wachstum von Fibroplasten fördernde Substanzen, wie Fibronectin, sowie infektionshemmende Arzneimittel enthalten kann, beschichtet wird.

3. Verfahren nach Anspruch 1, dadurch gekennzeichnet, daß die Fibrinogenkomponente und die Thrombinkomponente einzeln oder gemeinsam in einem organischen Lösungsmittel, das geringe Mengen an Wasser enthalten kann, suspendiert, nötigenfalls gemischt, auf eine oder beide Oberflächen des Kollagenträgers aufgetragen werden, worauf das Lösungsmittel verdampft wird.

4. Verfahren nach Anspruch 3, dadurch gekennzeichnet, daß die Suspension aufgesprüht wird.

5. Verfahren nach den Ansprüchen 1 bis 4, dadurch gekennzeichnet, daß als Kollagenträger ein Kollagenschaum eingesetzt wird.

6. Verfahren nach den Ansprüchen 1 bis 5, dadurch gekennzeichnet, daß Fibrinogenpartikel in einer Menge von 0,05 bis 20 mg/cm$^2$ des zu beschichtenden Materials eingesetzt werden.

7. Verfahren nach Anspruch 6, dadurch gekennzeichnet, daß Fibrinogenpartikel in einer Menge von 0,5 bis 5 mg/cm$^2$ des zu beschichtenden Materials eingesetzt werden.

8. Verfahren nach den Ansprüchen 1 bis 7, dadurch gekennzeichnet, daß Thrombin oder thrombinfreisetzende Partikel in einer Menge von 1 μg bis 5 mg/cm$^2$ eingesetzt werden.

9. Verfahren nach Anspruch 8, dadurch gekennzeichnet, daß Thrombin oder thrombinfreisetzende Partikel in einer Menge von 50 μg bis 1 mg/cm$^2$ eingesetzt werden.

**Claims** (for the contracting States : BE, CH, DE, FR, GB, IT, LI, LU, NL, SE)

1. Material for sealing and healing of wounds, which essentially consists of collagen and substances which cause blood clotting, and is obtainable by coating a collagen carrier base, in the presence of an organic solvent which can contain small quantities of water, on one face or all faces with a mixture of a fibrinogen component containing fibrinogen, factor XIII-containing fibrinogen or mixtures thereof, and with a thrombin component, containing thrombin, substances which liberate thrombin in the presence of body fluid, or mixtures of such substances, which mixture may moreover contain conventional additives, such as calcium ions, protease inhibitors, heparin antagonists, substances, such as fibronectin, which promote the infiltration and growth of fibroblasts, as well as anti-infection medicaments, and by subsequent evaporation of the solvent.

2. Material for sealing and healing of wounds, which essentially consists of collagen and substances which cause blood clotting, and is obtainable by coating a collagen carrier base, moistened with a small quantity of water, on one face or all faces with a mixture of a solid fibrinogen component containing fibrinogen, factor XIII-containing fibrinogen or mixtures thereof, with a solid thrombin component, containing thrombin, substances which Liberate thrombin in the presence of body fluid, or mixtures of such substances, which mixture may moreover contain conventional additives, such as calcium ions, protease inhibitors, heparin antagonists, substances, such as fibronectin, which promote the infiltration and growth of fibroblasts, as well as anti-infection medicaments.

3. Material according to Claims 1 and 2, characterised in that the collagen carrier is a collagen foam.

4. Material according to Claims 1 to 3, characterised in that the amount of fibrinogen particles is 0.05 to 20 mg/cm$^2$.

5. Material according to Claim 4, characterised in that the amount of fibrinogen particles is 0.5 to 5 mg/cm$^2$.

6. Material according to Claims 1 to 5, characterised in that the amount of thrombin or thrombin-liberating particles is 1 μg to 5 mg/cm$^2$.

7. Material according to Claim 6, characterised in that the amount of thrombin or thrombin-liberating particles is 50 µg to 1 mg/cm².

8. Process for the preparation of a material for sealing and healing wounds, according to Claims 1, 3, 4, 5, 6 and 7, characterised in that a collagen carrier base is coated in the presence of an organic solvent which can contain small quantities of water, on one face or all faces with a mixture of a fibrinogen component containing fibrinogen, factor XIII-containing fibrinogen or mixtures thereof, and with a thrombin component, containing thrombin, substances which liberate thrombin in the presence of body fluid, or mixtures of such substances, which mixture may moreover contain conventional additives, such as calcium ions, protease inhibitors, heparin antagonists, substances, such as fibronectin, which promote the infiltration and growth of fibroblasts, as well as anti-infection medicaments, and the solvent is subsequently evaporated.

9. Process for the preparation of a material for sealing and healing wounds, according to Claims 2 to 7, characterised in that a collagen carrier base, moistened with a small quantity of water, is coated on one face or all faces with a mixture of a solid fibrinogen component containing fibrinogen, factor XIII-containing fibrinogen or mixtures thereof, a solid thrombin component, containing thrombin, substances which liberate thrombin in the presence of body fluid, or mixtures of such substances, which mixture may moreover contain conventional additives, such as calcium ions, protease inhibitors, heparin antagonists, substances, such as fibronectin, which promote the infiltration and growth of fibroblasts, as well as anti-infection medicaments.

10. Process according to Claim 8, characterised in that the fibrinogen component and the thrombin component are suspended, individually or conjointly, in an organic solvent which can contain small quantities of water, are mixed if necessary and are applied to one or both faces of the collagen carrier base, after which the solvent is evaporated.

11. Process according to Claim 10, characterised in that the suspension is applied by spraying.

## Claims (for the Contracting State/AT)

1. A material for sealing and healing of wounds, which contains collagen and substances which cause blood clotting, and consists of a collagen carrier base, which is coated on one face or all faces with a mixture of a fibrinogen component containing fibrinogen and/or factor XIII-containing fibrinogen, and a thrombin component, containing thrombin and/or substances which liberate thrombin in the presence of body fluid, which mixture may moreover contain conventional additives, such as calcium ions, protease inhibitors, heparin antagonists, substances, such as fibronectin, which promote the infiltration and growth of fibroblasts, as well as anti-infection medicaments.

2. A material as claimed in claim 1, wherein the collagen carrier is à collagen foam.

3. A material as claimed in claims 1 and 2, wherein the amount of fibrinogen particles is 0.05 to 20 mg/cm².

4. A material as claimed in claims 1 to 3, wherein the amount of thrombin or thrombin-liberating particles is 1 µg to 5 mg/cm².

5. A process for the preparation of the material as claimed in claims 1 to 4, wherein the mixture of fibrinogen component, containing fibrinogen and/or factor XIII-containing fibrinogen and the thrombin component, containing thrombin and substances which liberate thrombin in the presence of body fluid, in the presence of a medium which consists at least predominantly of an organic solvent, is applied to one face or all faces of the surface of the collagen carrier, and the solvent is evaporated.

6. A process as claimed in claim 5, wherein the fibrinogen component and the thrombin component are suspended, individually or conjointly, in an organic solvent, are mixed if necessary and are applied to one face of the collagen carrier, after which the solvent is evaporated.

7. A process as claimed in claim 6, wherein the suspension is applied by spraying.

8. A process as claimed in claim 5, wherein the fibrinogen component and the thrombin component are applied, in solid form, to a collagen carrier which has beforehand been moistened with the medium consisting predominantly of an organic solvent.

9. A modification of the process as claimed in claim 7, wherein, before the fibrinogen component and the thrombin component are applied, in solid form, the collagen carrier is moistened with a very small amount of water.

**Revendications** (pour les Etats contractants : BE, CH, DE, FR, GB, IT, LI, LU, NL, SE)

1. Matériau pour assurer la fermeture étanche et la guérison de plaies, constitué principalement par du collagène et des substances provoquant la coagulation du sang, obtenu par l'application, sur une face ou sur toutes les faces d'un support en collagène, en présence d'un solvant organique qui peut contenir de petites quantités d'eau, d'un revêtement formé par un mélange d'un constituant fibrinogène, contenant du fibrinogène, du fibrinogène contenant le facteur XIII ou des mélanges de ceux-ci, et d'un constituant thrombine contenant de la thrombine, des substances libérant de la thrombine en présence des liquides du corps ou des mélanges de celles-ci, et pouvant contenir en outre les additifs usuels comme des ions calcium, des inhibiteurs de protéase, des antagonistes de l'héparine, des substances favorisant la formation et le développement de fibroplastes, comme la fibronectine, ainsi que des médicaments anti-infectieux, puis évaporation du solvant.

2. Matériau pour assurer la fermeture étanche et la guérison de plaies, constitué principalement par du collagène et des substances provoquant la coagulation du sang, pouvant être obtenu par l'application, sur une face ou sur toutes les faces d'un support en collagène humidifié avec une petite quantité d'eau, d'un revêtement formé par un mélange d'un constituant fibrinogène solide, contenant du fibrinogène, du fibrinogène contenant le facteur XIII ou des mélanges de ceux-ci, un constituant thrombine solide contenant de la thrombine, des substances libérant de la thrombine en présence des liquides du corps ou des mélanges de celles-ci, et pouvant contenir en outre les additifs usuels comme des ions calcium, des inhibiteurs de protéase, des antagonistes de l'héparine, des substances favorisant la formation et le développement de fibroplastes comme la fibronectine, ainsi que des médicaments anti-infectieux.

3. Matériau suivant la revendication 1 ou 2, caractérisé en ce que le support en collagène est une mousse de collagène.

4. Matériau suivant l'une quelconque des revendications 1 à 3, caractérisé en ce qu'il contient des particules de fibrinogène en une quantité allant de 0,05 à 20 mg/cm².

5. Matériau suivant la revendication 4, caractérisé en ce qu'il contient des particules de fibrinogène en une quantité allant de 0,5 à 5 mg/cm².

6. Matériau suivant l'une quelconque des revendications 1 à 5, caractérisé en ce qu'il contient de la thrombine ou des particules libérant de la thrombine en une quantité allant de 1 µg à 5 mg/cm².

7. Matériau suivant la revendication 6, caractérisé en ce qu'il contient de la thrombine ou des particules libérant de la thrombine en une quantité allant de 50 µg à 1 mg/cm².

8. Procédé pour la fabrication d'un matériau pour la guérison et la fermeture étanche de plaies suivant les revendications 1, 3, 4, 5, 6 et 7, caractérisé en ce qu'on applique sur un support en collagène, sur une face ou sur toutes les faces, en présence d'un solvant organique qui peut contenir de petites quantités d'eau, un revêtement formé par un mélange d'un constituant fibrinogène, contenant du fibrinogène, du fibrinogène contenant le facteur XIII ou des mélanges de ceux-ci, et un constituant thrombine, contenant de la thrombine, des substances libérant de la thrombine en présence des liquides du corps ou des mélanges de celles-ci, et pouvant contenir en outre les additifs usuels comme des ions calcium, des inhibiteurs de protéase, des antagonistes de l'héparine, des substances favorisant la formation et le développement de fibroplastes comme la fibronectine, ainsi que des médicaments anti-infectieux, puis on élimine le solvant par évaporation.

9. Procédé pour la fabrication d'un matériau pour la guérison et la fermeture étanche de plaies suivant l'une quelconque des revendications 2 à 7, caractérisé en ce qu'on applique sur un support en collagène humidifié avec une petite quantité d'eau, sur une face ou sur toutes les faces, un revêtement formé par un mélange d'un constituant fibrinogène solide contenant du fibrinogène, du fibrinogène contenant le facteur XIII ou des mélanges de ceux-ci, un constituant thrombine solide contenant de la thrombine, des substances libérant de la thrombine en présence des liquides du corps ou des mélanges de celles-ci, et pouvant contenir en outre les additifs usuels comme des ions calcium, des inhibiteurs de protéase, des antagonistes de l'héparine, des substances favorisant la formation et le développement de fibroplastes comme la fibronectine, ainsi que des médicaments anti-infectieux.

10. Procédé suivant la revendication 8, caractérisé en ce qu'on met le constituant fibrinogène et le constituant thrombine, individuellement ou conjointement, en suspension dans un solvant organique qui peut contenir de petites quantités d'eau, si nécessaire on mélange, on applique sur une face ou sur les deux faces d'un support en collagène, après quoi on élimine le solvant par évaporation.

11. Procédé suivant la revendication 10, caractérisé en ce qu'on applique la suspension par projection ou pulvérisation.

**Revendications** (pour l'Etat contractant AT)

1. Procédé pour la fabrication d'un matériau pour pansements contenant du collagène et des substances provoquant la coagulation du sang, caractérisé en ce qu'on applique sur un support en collagène, sur une face ou sur toutes les faces, en présence d'un solvant organique qui peut contenir de petites quantités d'eau, un revêtement formé par un mélange d'un constituant fibrinogène contenant du fibrinogène, du fibrinogène contenant le facteur XIII ou des mélanges de ceux-ci, un constituant thrombine contenant de la thrombine, des substances libérant de la thrombine en présence des liquides du corps ou des mélanges de celles-ci, et pouvant contenir en outre les additifs usuels comme des ions calcium, des inhibiteurs de protéase, des antagonistes de l'héparine, des substances favorisant la formation et le développement de fibroplastes comme la fibronectine, ainsi que des médicaments anti-infectieux, puis on élimine le solvant par évaporation.

2. Procédé pour la fabrication d'un matériau pour pansements contenant du collagène et des substances provoquant la coagulation du sang, caractérisé en ce qu'on applique sur un support en collagène humidifié avec une petite quantité d'eau, sur une face ou sur toutes les faces, un revêtement formé par un mélange d'un constituant fibrinogène solide contenant du fibrinogène, du fibrinogène contenant le facteur XIII ou des mélanges de ceux-ci, un constituant thrombine solide contenant de la thrombine, des substances libérant de la thrombine en présence des liquides du corps ou des mélanges de celles-ci, et pouvant contenir en outre les additifs usuels comme des ions calcium, des inhibiteurs de protéase, des antagonistes de l'héparine, des substances favorisant la formation et le développement de fibroplastes comme la fibronectine, ainsi que des médicaments anti-infectieux.

3. Procédé suivant la revendication 1, caractérisé en ce qu'on met le constituant fibrinogène et le constituant thrombine, individuellement ou conjointement, en suspension dans un solvant organique qui peut contenir de petites quantités d'eau, si nécessaire on mélange, on applique sur une face ou sur les deux faces d'un support en collagène, après quoi on élimine le solvant par évaporation.

4. Procédé suivant la revendication 3, caractérisé en ce qu'on applique la suspension par projection ou pulvérisation.

5. Procédé suivant l'une quelconque des revendications 1 à 4, caractérisé en ce qu'on utilise comme support en collagène une mousse de collagène.

6. Procédé suivant l'une quelconque des revendications 1 à 5, caractérisé en ce qu'on utilise des particules de fibrinogène en une quantité représentant de 0,05 à 20 mg/cm² du matériau à recouvrir.

7. Procédé suivant la revendication 6, caractérisé en ce qu'on utilise des particules de fibrinogène en une quantité représentant de 0,5 à 5 mg/cm² du matériau à recouvrir.

8. Procédé suivant l'une quelconque des revendications 1 à 7, caractérisé en ce qu'on utilise de la thrombine ou des particules libérant de la thrombine en une quantité allant de 1 μg à 5 mg/cm².

9. Procédé suivant la revendication 8, caractérisé en ce qu'on utilise de la thrombine ou des particules libérant de la thrombine en une quantité allant de 50 μg à 1 mg/cm².